(19) Europäisches Patentamt
European Patent Office
Office européen des brevets

(11) **EP 4 545 146 A1**

(12) **EUROPEAN PATENT APPLICATION**
published in accordance with Art. 153(4) EPC

(43) Date of publication:
**30.04.2025 Bulletin 2025/18**

(21) Application number: 23831029.6

(22) Date of filing: **09.06.2023**

(51) International Patent Classification (IPC):
*A61Q 1/00* (2006.01)     *A61Q 17/04* (2006.01)
*A61Q 19/00* (2006.01)     *A61K 8/04* (2006.01)
*A61K 8/19* (2006.01)      *A61K 8/34* (2006.01)
*A61K 8/893* (2006.01)

(52) Cooperative Patent Classification (CPC):
**A61K 8/04; A61K 8/19; A61K 8/34; A61K 8/893;**
**A61Q 1/00; A61Q 17/04; A61Q 19/00**

(86) International application number:
**PCT/JP2023/021486**

(87) International publication number:
**WO 2024/004579 (04.01.2024 Gazette 2024/01)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **27.06.2022 JP 2022102778**

(71) Applicant: **SHIN-ETSU CHEMICAL CO., LTD.**
**Tokyo 1000005 (JP)**

(72) Inventors:
• **MIYAUCHI Masaru**
  **Tokyo 100-0005 (JP)**
• **KONISHI Masayuki**
  **Tokyo 100-0005 (JP)**

(74) Representative: **Mewburn Ellis LLP**
**Aurora Building**
**Counterslip**
**Bristol BS1 6BX (GB)**

(54) **DISPERSION AND COSMETIC CONTAINING SAME**

(57)     A dispersion of fine inorganic particles comprising the following (a) to (c) components:
(a) 10-70 mass% the inorganic fine particles, which are hydrophobized inorganic fine particles having a number-average primary-particle diameter, as determined by analyzing images on a transmission electron photomicrograph, of 10-200 nm;
(b) 1.0-30 mass% aqueous component having one or more alcoholic hydroxyl groups; and
(c) 1.0-20 mass% polyglycerin-modified silicone. The (c) component has dissolved in the (b) component. The dispersion has excellent dispersibility in aqueous media and high dispersion stability.

**Description**

TECHNICAL FIELD

This invention relates to a dispersion comprising a fine particle of inorganic powder and a cosmetic composition having the dispersion blended therein.

BACKGROUND ART

In sunscreen cosmetics, inorganic powders such as titanium oxide and zinc oxide are commonly used in fine particle form for the purposes of improving transparency and enhancing the UV-shielding effect. The inorganic powders, however, have the propensity that as the surface area becomes larger, the interaction among particles becomes stronger and particles are more likely to agglomerate.

Dispersions are prepared from fine particle of inorganic powders for the purposes of facilitating blending of powders into cosmetics and improving transparency and UV-shielding effect. Thus far, numerous studies have been made on the technique of dispersing highly agglomerative powders (see Patent Document 1). It is known from Patent Document 2 that among others, polyglycerin-modified silicone is fully helpful to disperse the above-mentioned powders. Patent Document 3 describes an O/W type cosmetic having a fine particle of inorganic powder dispersed in an oil phase. However, no studies have been made on the dispersion of highly agglomerative powder in an aqueous medium with the aid of polyglycerin-modified silicone.

On the other hand, Patent Document 4 describes a surface treated powder which is coated or treated with titanium oxide or zinc oxide so that the powder may be more dispersible in water. When the treated powder is blended in a cosmetic, however, there arise problems with respect to transparency, spreading extent on coating, and feeling on use.

Also, Patent Document 5 discloses a dispersion containing a nonionic surfactant, which still fails to gain satisfactory dispersibility.

Under the circumstances, there exists the need for a dispersion having more improved dispersibility and providing a pleasant feeling on use when blended in cosmetics.

PRIOR ART DOCUMENTS

PATENT DOCUMENTS

Patent Document 1: JP-A 2006-001886
Patent Document 2: WO 2016/178380
Patent Document 3: JP-A 2014-201569
Patent Document 4: JP-A 2007-016111
Patent Document 5: JP-A H07-247119

SUMMARY OF INVENTION

TECHNICAL PROBLEM

An object of the invention, which has been made under the above-mentioned circumstances, is to provide a fine particle of inorganic powder dispersion having improved dispersibility in aqueous media and high dispersion stability. Another object is to provide a cosmetic composition having the dispersion blended therein and exhibiting high stability and improved transparency, non-sticky pleasant feeling on use, and water resistance.

SOLUTION TO PROBLEM

Making extensive investigations to attain the above object, the inventors have found that a dispersion comprising components (a) to (c):

(a) 10 to 70% by weight of a hydrophobized fine particle of inorganic powder having a number average primary particle diameter of 10 to 200 nm, as determined by image analysis of a transmission electron photomicrograph,
(b) 1.0 to 30% by weight of an aqueous component having at least one alcoholic hydroxy group, and
(c) 1.0 to 20% by weight of a polyglycerin-modified silicone,

component (c) being dissolvable in component (b), is a stable dispersion having a low viscosity and a little change with time of viscosity. When the dispersion is blended in a cosmetic composition, the cosmetic composition gives a pleasant feeling on use without giving rise to problems like viscosity lowering and gelation. The invention is predicated on this finding.

Accordingly, the invention provides a dispersion and cosmetic composition as defined below.

1. A dispersion comprising components (a) to (c):

(a) 10 to 70% by weight of a hydrophobized fine particle of inorganic powder having a number average primary particle diameter of 10 to 200 nm, as determined by image analysis of a transmission electron photomicrograph,
(b) 1.0 to 30% by weight of an aqueous component having at least one alcoholic hydroxy group, and
(c) 1.0 to 20% by weight of a polyglycerin-modified silicone which dissolves in component (b).

2. The dispersion of 1 wherein the fine particle of inorganic powder as component (a) has a UV-shielding effect.
3. The dispersion of 2 wherein the fine particle of inorganic powder as component (a) is titanium oxide.
4. The dispersion of 1 or 2 wherein component (a) is a fine particle of inorganic powder which has been hydrophobized with at least one agent selected from stearic acid, isostearic acid and triethoxycaprylylsilane.
5. The dispersion of any one of 1 to 4 wherein component (b) is a compound having two alcoholic hydroxy groups in the molecule.
6. The dispersion of any one of 1 to 4 wherein component (c) is polyglyceryl-3 disiloxane dimethicone.
7. The dispersion of any one of 1 to 6 wherein the ratio of the amount of component (c) to the amount of component (b), (c)/(b), is from 0.35/1 to 1.0/1.
8. The dispersion of any one of 1 to 7, further comprising (d) water.
9. The dispersion of 8 wherein the content of component (d) is 8 to 82% by weight of the dispersion.
10. A cosmetic composition comprising the dispersion of any one of 1 to 9.

ADVANTAGEOUS EFFECTS OF INVENTION

According to the invention, there is provided a dispersion having satisfactory dispersibility in aqueous media and high dispersion stability. When the dispersion is blended in a cosmetic composition, the dispersion is easy to admit and the cosmetic composition has transparency, a pleasant feeling on use, and water resistance. Even from fine particle of titanium oxide which is difficult to disperse, a highly stable dispersion is obtainable.

DESCRIPTION OF EMBODIMENTS

Now the invention is described in detail. Herein, the name of ingredients is sometimes expressed by the Japanese labeling name or the International Nomenclature for Cosmetic Ingredients (INCI). When the Japanese labeling name is identical with the INCI, sometimes either one is omitted.

[Component (a)]

Component (a) is a hydrophobized fine particle of inorganic powder. The fine particle of inorganic powder may be any of powders having a UV-shielding effect and used as a UV-scattering agent as long as it is commonly blended in cosmetics. The powders may be used alone or in admixture of two or more. Inter alia, one or more metal oxides selected from titanium oxide, zinc oxide, and cerium oxide are preferred. The metal oxide may also be a composite powder of two or more of titanium oxide, zinc oxide, and cerium oxide, or one or more thereof with another powder.

Component (a) should have a number average primary particle diameter of 10 to 200 nm, preferably up to 150 nm, as determined by image analysis of a transmission electron photomicrograph. If the particle diameter is more than 200 nm, the UV-protecting function lowers and white spots are left. If the particle diameter is less than 10 nm, dryness may become stronger, adversely affecting the feeling on use. The average primary particle diameter of component (a) can be measured from a transmission electron photomicrograph. When particles are not spherical, the average primary particle diameter is given by the average of breadths of particles. The fine particle of inorganic powder may be of spindle, needle, bunch, strip, substantial sphere, and rod shapes.

The fine particle of inorganic powder may have been surface-treated with silica, hydrous silica, alumina or aluminum hydroxide, prior to the hydrophobic treatment, for the purpose of weakening agglomeration or reducing the activity of the powder. If there is concern about the inhibition of swelling of a water-soluble polymer or a loss of water resistance after blending of the powder in cosmetics, a composite powder which has not been surface-treated with alumina or aluminum hydroxide is preferred. If there is concern about a loss of water resistance, a composite powder which has not been surface-treated with silica or hydrous silica is preferred.

The hydrophobizing agent for the fine particle of inorganic powder is not particularly limited as long it is a well-known treating agent commonly used in cosmetics. Suitable treating agents include silanes and silylating agents such as caprylylsilane (AES-3083 by Shin-Etsu Chemical Co., Ltd.); silicone oils such as dimethyl silicone (KF-96AK series by Shin-Etsu Chemical Co., Ltd.), methylhydrogen polysiloxane (KF-99P and KF-9901 by Shin-Etsu Chemical Co., Ltd.), and silicone branched silicone treating agents (KF-9908 and KF-9909 by Shin-Etsu Chemical Co., Ltd.); waxes, paraffins, organic fluorine compounds such as perfluoroalkyl phosphates, surfactants, amino acids such as N-acylglutamic acid, and metal soaps such as aluminum stearate and magnesium myristate. In particular, an agent containing at least one of stearic acid, isostearic acid, and triethoxycaprylylsilane is preferred because of satisfactory treatment and good dispersion with component (c). The hydrophobizing treatment is not particularly limited. The treatment can be performed by any well-known methods, for example, wet treatment, dry treatment and gas phase methods.

As the surface-treated fine particle of inorganic powder, any commercially available powder may be used. For example, the surface-treated fine particle of titanium oxide is commercially available under the trade name of MT-01, 02, 0500TS, 100Z, 100TV, 100SAS, 150EX, 200ST, 500SAM, 505SAS, 700Z, and 700BS (Tayca Corp.), ST-455, 455WS, 457ECS, 457SA, 495M, and 455F (Titan Kogyo, Ltd.), STR-100A-LP, 100C-LP, 100W-LP, 100C-LF, and 40-LP (Sakai Chemical Industry Co., Ltd.). The surface-treated fine particle of zinc oxide is commercially available under the trade name of MZ-150, 200, 300, 306X, 500HP, 505T, 506X, MZY-203S, 210M3S, TMZ-HA1, and MZX-5080TS (Tayca Corp.), and FZO-50 (Ishihara Sangyo Kaisha, Ltd.).

The content of component (a) is 10 to 70% by weight, preferably 20 to 70% by weight of the dispersion in view of versatility. Particularly when water (d) is blended, the content of component (a) is preferably 10 to 55% by weight, more preferably 30 to 50% by weight of the dispersion. If the content of component (a) is less than 10% by weight, no satisfactory UV-shielding effect is obtainable. If the content exceeds 70% by weight, there is a risk that spreadability on use is aggravated or the dispersion loses age stability and experiences a viscosity buildup.

[Component (b)]

Component (b) is an aqueous component having at least one alcoholic hydroxy group, that is, a component which dissolves in water at 25°C, which may be used alone or in admixture of two or more. Examples include ethanol (labeling name, INCI: Alcohol), isopropanol (labeling name, INCI: Isopropyl Alcohol), lower alcohols of preferably 2 to 5 carbon atoms, sorbitol (INCI), maltose (INCI), xylitol (INCI), glucose (INCI), glyceryl glucoside (INCI), sodium chondroitin sulfate (labeling name, INCI: Sodium Chondroitin Sulfate), Methyl Gluceth-10 (INCI), Methyl Gluceth-20 (INCI), hyaluronic acid, and sucrose alcohols such as phosphatidyl glycerol and phosphatidyl inositol. Also included are polyhydric alcohols such as BG (labeling name, INCI: Butylene Glycol), PG (labeling name, INCI: Propylene Glycol), DPG (labeling name, INCI: Dipropylene Glycol), pentylene glycol (INCI), 1,10-decandiol (INCI), octanediol (INCI), 1,2-hexanediol (INCI), erythritol (INCI), glycerin (INCI), diglycerin (INCI), and polyethylene glycol. Of these, polyhydric alcohols such as BG (butylene glycol), DPG (dipropylene glycol), and glycerin are preferred because they dissolve in water in any proportion and in view of feeling on use and versatility as cosmetic ingredients. Especially, glycols having two alcoholic hydroxy groups in the molecule are more preferred.

The content of component (b) is 1.0 to 30% by weight, preferably 1.5 to 25% by weight, more preferably 5 to 25% by weight, even more preferably 5.0 to 15% by weight of the dispersion. If the content of component (b) is less than 1.0% by weight, the dispersion becomes less stable. If the content exceeds 30% by weight, a cosmetic composition gives a sticky feeling on use, and component (b) prevents component (c) from orienting toward component (a), leading to a high viscosity or detracting from dispersibility in aqueous media. Component (b), when blended, helps component (c) (to be described below) uniformly orient on the surface of component (a).

[Component (c)]

Component (c) is a polyglycerin-modified silicone which dissolves in component (b). As used herein, the phrase that component (c) "dissolves in component (b)" means that after a mixture of component (c) with component (b) in a concentration of 20% by weight is allowed to stand at 25°C for 1 hour, component (c) "dissolves" when the mixture remains transparent to semi-transparent without any boundary whereas component (c) "does not dissolve" when the mixture becomes white turbid or separates into two layers. With respect to transparency, the mixture is judged transparent to semi-transparent when the total light transmission through a cell of 1 cm thick which is filled with the mixture, as measured according to JIS K7361-1: 1997, is 50% or more.

The polyglycerin-modified silicone has the chemical structure that silicone chain as the backbone may be modified with polyglycerin either in blocks or in branched form. From the aspect of maintaining the uniform dispersibility of component (a) in a cosmetic composition, the polyglycerin-modified silicone of branched form is preferred. The silicone backbone may include branched chains such as silicone chains. Specifically, polyglyceryl-3 disiloxane dimethicone (INCI) is exemplary. As the commercially available product, KF-6100 (Shin-Etsu Chemical Co., Ltd.) is exemplary. It is noted that those silicones which belong to the polyglycerin-modified silicone, but do not dissolve in component (b), for example, polyglyceryl-3 polydimethylsiloxyethyl dimethicone (labeling name, INCI: Polyglyceryl-3 Polydimethylsiloxyethyl Dimethicone), and lauryl polyglyceryl-3 polydimethylsiloxyethyl dimethicone (labeling name, INCI: Lauryl Polyglyceryl-3 Polydimethylsiloxyethyl Dimethicone), are not included in component (c).

The content of component (c) is 1.0 to 20% by weight, preferably 3 to 20% by weight, more preferably 5 to 15% by weight, even more preferably 7 to 12% by weight of the dispersion. If the content of component (c) is less than 1.0% by weight, the dispersion becomes less stable. If the content exceeds 20% by weight, there is a likelihood that component (c) further orients to component (c) having oriented toward component (a), or component (c) having oriented toward component (a) is peeled off by component (c) not having oriented toward component (a), leading to a dispersion having a high viscosity or adversely affecting the dispersibility in aqueous media.

In a preferred embodiment, the ratio of the amount of component (c) to the amount of component (b), represented by (c)/(b), is from 0.35/1 to 1.0/1. The ratio is more preferably from 0.4 to 0.9, even more preferably from 0.7 to 0.9 from the aspects of dispersibility and water resistance. If the ratio is less than 0.35, there is a possibility that component (b) prevents component (c) from orienting toward component (a), leading to a high viscosity or detracting from stability. If the ratio exceeds 1.0, there is a likelihood that component (c) is not fully dispersed in a dispersing medium or does not orient toward component (a), leading to a dispersion having a high viscosity or adversely affecting stability.

[Component (d)]

In the invention, water may be blended as component (d). Examples of water used herein include ion exchanged water, distilled water, deionized water, purified water defined in the Japanese Pharmacopoeia, spring water, and deep ocean water. According to the invention, a stable dispersion having a low viscosity is obtained even when component (d) is blended.

When used, the amount of component (d) blended is preferably 8 to 82% by weight, more preferably 28 to 72% by weight, even more preferably 32 to 59% by weight of the dispersion. Even when the amount is less than 8% by weight, a stable dispersion is obtainable, but such a small amount is difficult to attain the object of blending component (d) to lower the viscosity. If the amount exceeds 82% by weight, a problem can arise in age stability.

When component (d) is blended, a defoamer may be added for the purpose of facilitating handling during preparation or filling. Examples of the defoamer include simethicone (INCI), dimethicone (INCI), salts such as sodium chloride (labeling name, INCI: Sodium Chloride), and polyether-modified silicone. Also, oils such as dimethicone may be of emulsion type (previously emulsified), and compounds such as simethicone may be of self-emulsion type (previously mixed with silicone surfactant). Of these, simethicone and sodium chloride are preferred from the aspect of foam breaking or foam suppression. Simethicone is most preferred from the aspect of affinity to cosmetic ingredients. The amount of the defoamer blended is preferably 0.0001 to 1% by weight, more preferably 0.0025 to 0.6% by weight, even more preferably 0.005 to 0.01% by weight of the overall dispersion.

When component (d) is not blended, the inventive dispersion becomes a slurry dispersion having such a high concentration that the dispersion may be amenable to transportation and formulation. On the other hand, blending of component (d) leads to a dispersion having a low viscosity and ease of handling.

Particularly when component (d) is blended, the dispersion preferably has an absolute viscosity at 25°C of 1 mPa·s to less than 6,000 mPa·s, more preferably 1 mPa·s to less than 1,000 mPa·s, even more preferably 1 mPa·s to less than 500 mPa·s. It is noted that the absolute viscosity is measured by a Brookfield viscometer according to the method of JIS K7117-1: 1999.

[Dispersion]

The dispersion of the invention is readily blended in aqueous media. There is obtained an aqueous dispersion which experiences temporary sedimentation, but turns uniformly dispersed as stirring is continued, in the dispersibility test described later in Example.

[Method of preparing dispersion]

The method and apparatus used when the inventive dispersion is prepared is not particularly limited, and any well-known method may be used. There may be used, for example, any agitators, mills, mixers, media agitating mills, rotation/revolution agitators, and dispersing machines such as Henschel mixers, ball mills, kneaders, planetary mixers, ribbon blenders, dispersing mixers, homo-mixers, jet mills, roll mills, and bead mills. Particularly from the aspect of mixing efficiency, the step of dispersing on a bead mill is preferred.

[Cosmetic composition]

While the dispersion of the invention may be used in a variety of applications, it is particularly useful as one ingredient for cosmetic compositions which are externally applied to the skin and hair.

The form that the cosmetic composition containing the inventive dispersion takes may be any of water-in-oil emulsion cosmetic, polyhydric alcohol-in-oil, oil-in-water emulsion cosmetic, aqueous cosmetic, and multi-phase emulsions such as W/O/W and O/W/O. Particularly when the dispersion is used in a oil-in-water emulsion cosmetic, the dispersion is easy to admit and a stable cosmetic composition having transparency, feeling on use, and water resistance is obtainable.

The form of the inventive cosmetic composition may be selected from among liquid, milky lotion, cream, solid, paste, gel, powder, pressed, multilayer, mousse, spray, stick, pencil and other forms. The multilayer form refers to a cosmetic composition which separates into two or more layers on static holding. It is filled in a container containing stainless steel balls, and used after shaking the container. Since the inventive dispersion has satisfactory dispersibility, the composition of such form can be readily re-dispersed. The composition of such form is named shaking type. It is a cosmetic composition giving a pleasant feeling on use because of easy stabilization, despite the need for shaking. Since the inventive cosmetic composition has satisfactory stability, it can also be used without separation into multiple layers. The spray form refers to a spraying cosmetic composition which is filled in a dispenser or aerosol container and used by spraying through a nozzle. The cosmetic composition filled in a dispenser container is sprayed as mist through the nozzle on the dispenser. The cosmetic composition is filled in the aerosol container together with a spraying agent. The spraying agent is not particularly limited and selected from, for example, various liquefied petroleum gases (LPG), dimethyl ether, nitrogen gas, and carbon dioxide gas. The spraying agent may be used alone or in admixture. Since the inventive dispersion has high dispersibility, it can also be used in such form.

The invention is applicable to a variety of cosmetic compositions, preferably cosmetic compositions externally applied to the skin such as skin care cosmetic compositions, make-up cosmetic compositions, antiperspirant cosmetic compositions, and UV-shielding cosmetic compositions and cosmetic compositions externally applied to the hair such as hair care cosmetic compositions. Exemplary skin care cosmetic compositions include toilet water, milky lotion, cream, cleansing agent, pack, oil liquid, massage cream, cosmetic liquid, cosmetic oil, detergent, deodorant, hand cream, lip cream, and anti-wrinkle agent. Exemplary makeup cosmetic compositions include makeup foundation, concealer, cosmetic powder, powder foundation, eye color, eye shadow, mascara, eye liner, eye blow, and lip stick. Exemplary antiperspirant cosmetic compositions include antiperspirants of roll-on, cream, solution, and stick types. Exemplary UV-protecting cosmetic compositions include sun-screen oil, sun-screen milky lotion, and sun-screen cream. Exemplary hair care cosmetic compositions include shampoo, rinse, treatment, and setting agent. Among these, the UV-protecting cosmetic compositions are most preferred.

The cosmetic composition of the invention may contain various components which are commonly used in conventional cosmetics as long as the benefits of the invention are not impaired. Suitable components include, for example, (1) oils, (2) aqueous components other than components (b) and (d), (3) surfactants other than component (c), (4) powders other than component (a), (5) compositions consisting of crosslinked organopolysiloxane and an oil which is liquid at room temperature, (6) film-forming agents, and (7) other additives. They may be used alone or in admixture of two or more.

(1) Oils

The oils may be volatile or non-volatile and solid, semisolid or liquid at room temperature (25°C). Examples include silicone oil, silicone wax, naturally occurring animal and plant oils and fats and semi-synthetic oils and fats, hydrocarbon oils, higher alcohols, fatty acids, ester oils, fluorinated oils, and UV absorbers.

• Silicone oil

Examples of the silicone oil include dimethicone (INCI), trisiloxane (INCI), alkyl-modified silicones such as methyl trimethicone (INCI), ethyl trisiloxane (INCI), ethyl methicone (INCI), and hexyl dimethicone (INCI), long chain alkyl-modified silicones such as caprylyl methicone (INCI), low to high viscosity straight or branched organopolysiloxanes such as phenyl trimethicone (INCI), diphenyl dimethicone (INCI), diphenylsiloxyphenyl trimethicone (INCI), tetraphenyl dimethyldisiloxane (INCI), and methylhydrogenpolysiloxane, cyclic organopolysiloxanes such as cyclotetrasiloxane (INCI), cyclopentasiloxane (INCI), and cyclohexasiloxane (INCI), amino-modified organopolysiloxanes such as amodimethicone (INCI) and aminopropyl dimethicone (INCI), pyrrolidone-modified organopolysiloxanes such as PCA dimethicone (INCI), defoamers such as simethicone (INCI), pyrrolidone carboxylic acid-modified organopolysiloxanes, silicone rubbers such as gum-like dimethylpolysiloxane having a high degree of polymerization, gum-like amino-modified organopolysiloxane, and gum-like dimethylsiloxane-methylphenylsiloxane copolymers, as well as solutions of low viscosity organopolysiloxanes such as silicone gum and rubber, amino acid-modified silicones, fluorine-modified silicones, silicone resins, and silicone resin solutions.

Commercially available examples of the silicone oil include KF-96L-1cs, KF-96L-1.5cs, KF-96L-2cs, KF-96A-6cs, KF-4422, KF-54, KF-54HV, KF-56A, and KF-995 by Shin-Etsu Chemical Co., Ltd.

• Solid oily component

When it is desired that the cosmetic composition be solidified, an oily component which is solid at 25°C is preferably blended. Examples of the oily component which is solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes such as carnauba wax (INCI: Copernicia Cefifera (Carnauba) Wax), sugar cane wax, candelilla wax (INCI: Euphorbia Cerifera (Candelilla) Wax), purified candelilla wax, rice wax, Japan wax, jojoba wax, capok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes such as beeswax, head, beef bone oil, lard (INCI: Lard), horse fat (INCI: Horse Fat), tallow, lanolin (INCI: Lanolin), insect wax, shellac wax and sperm wax; semi-synthetic waxes such as lanolin ester, lanolin fatty acid esters, and beeswax acid esters; hydrogenated oils such as hydrogenated castor oil and hydrogenated coconut oil; hydrocarbon waxes such as solid paraffin, polyethylene, ceresin, ozokerite, and microcrystalline wax; wax esters such as synthetic beeswax; amic acid stearyl alcohols such as dioctyldodecyl lauroylglutaminate, dioctyldodecyl lauroylglutaminate, and dioctyldodecyl lauroylglutaminate; fatty acids such as stearic acid and behenic acid; and silicone waxes such as acrylate silicone resins in the form of acrylate silicone graft or block copolymers (acrylate silicone graft copolymers: KP-561P, 562P by Shin-Etsu Chemical Co., Ltd.) or derivatives thereof. Any one or more compounds selected from the foregoing is preferred.

• Natural animal and plant oils and semi-synthetic oils

Examples of the natural animal and plant oils and semi-synthetic oils include naturally occurring plant oils such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (Calfornia Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), wheat germ oil (labeling name, INCI: Triticum Vulgare (Wheat) Germ Oil); germ oils such as rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), sufflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), tea seed oil (labeling name, INCI: Camellia Japonica Seed Oil), evening primrose oil (labeling name, INCI: Oenothera Biennis (Evening Primrose) Oil), rapeseed oil (labeling name), corn germ oil (labeling name, INCI: Zea Mays (Corn) Germ Oil), wheat germ oil (labeling name, INCI: Triticum Vulgare (Wheat) Germ Oil); natural plant oils such as persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Archis Hypogaea (Peanut) Oil); natural animal oils such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg oil (labeling name, INCI: Egg Oil); and semi-synthetic oils and fats such as hydrogenated coconut oil (labeling name, INCI: Hydrogenated Coconut Oil) and liquid lanolin (labeling name, INCI: Lanolin Oil).

• Hydrocarbon oil

The hydrocarbon oils include straight or branched hydrocarbon oils while they may be either volatile or nonvolatile. Examples of the hydrocarbon oil include olefin oligomers (INCI), isoparaffins such as (C13, C14) isoparaffins (INCI), isododecane (INCI), undecane (INCI), dodecane (INCI), isohexadecane (INCI), hydrogenated polyisobutene (labeling name, INCI: Hydrogenated Polyisobutene), squalane (INCI), mineral oil (INCI), and alkanes such as coconut alkane (INCI) and (C13-15) alkane (INCI).

• Higher alcohol

The higher alcohols include, for example, alcohols of 6 or more carbon atoms, preferably 10 to 30 carbon atoms. Examples of the higher alcohol include lauryl alcohol (INCI), myristyl alcohol (INCI), palmityl alcohol (INCI), stearyl alcohol (INCI), behenyl alcohol (INCI), oleyl alcohol (INCI), isostearyl alcohol (INCI), octyl dodecanol (INCI), cholesterol (INCI), phytosterol (INCI), and batyl alcohol (INCI).

• Ester oil

Examples of the ester oil include diisobutyl adipate (labeling name, INCI: Diisobutyl Adipate), dihexyldecyl adipate (labeling name), diheptylundecyl adipate (labeling name, INCI: Diheptylundecyl Adipate), n-alkylglycol monoisostearates such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and coco-alkyl caprylate-caprate (labeling name, INCI: Coco-Caprylate-Caprate).

Glyceride oils are also included in the ester oils, such as triethylhexanoin (INCI), glyceryl tri(caprylate/caprate) (labeling name, INCI: Caprylic/Capric Triglyceride), cocoglyceryl (INCI), triglyceryl caprylate/caprate/succinate (labeling name, INCI: Caprylic/Capric/Succinic Triglyceride), and caprylic/capric glycerides (labeling name, INCI: Caprylic/Capric Glycerides).

• Fluorochemical oils

Examples of the fluorochemical oil include perfluorodecalin (INCI), perfluorononyl dimethicone (INCI), and perfluoromethylcyclopentane (INCI).

• UV absorbers

Suitable UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), homosalate (INCI), octocrylene (INCI), t-butyl methoxydibenzoylmethane (labeling name, INCI: Butyl Methoxydibenzoylmethane), ethyl hexyl salicylate (labeling name, INCI: Ethyl Hexyl Salicylate), diethylaminohydroxybenzoyl hexyl benzoate (labeling name, INCI: Diethylamino Hydroxybenzoyl Hexyl Benzoate), polysilicone-15 (INCI), dimethoxybenzylidene dioxoimidazolidine octyl propionate (labeling name, INCI: Ethylhexyl Dimethoxybenzylidene Dioxoimidazolidine Propionate), oxybenzone-2 (labeling name, INCI: Benzophenone-2), terephthalylidene dicamphor sulfonic acid (labeling name, INCI: Terephthalylidene Dicamphor Sulfonic Acid), ethylhexyl triazone (INCI), methylbis(trimethylsiloxy)silyl isopentyl trimethoxycinnamate (labeling name, INCI: Isopentyl Trimethoxycinnamate Trisiloxane), drometrizole trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), bisethylhexyloxyphenol methoxyphenyltriazine (INCI), phenylbenzimidazole sulfonic acid (labeling name, INCI: Phenylbenzimidazole Sulfonic Acid), methylene bisbenzotriazolyl tetramethylbutylphenol (INCI), glyceryl ethylhexanoate dimethoxycinnamate (labeling name, INCI: Glyceryl Ethylhexanoate Dimethoxycinnamate), glyceryl PABA (INCI), methyl diisopropylcinnamate (labeling name, INCI: Diisopropyl Methyl Cinnamate), cinoxate (INCI), ethylhexyl dimethoxybenzylidene dioxoimidazolidine propionate (labeling name, INCI: Ethylhexyl Dimethoxybenzylidene Dioxoimidazolidine Propionate), etc.

(2) Aqueous components other than components (b) and (d)

The aqueous components are not particularly limited as long as they are other than components (b) and (d) and commonly blended in cosmetics. Examples include humectants such as betaine (INCI), PCA-Na (labeling name, INCI: Sodium PCA), egg yolk lecithin, soybean lecithin, phosphatidylcholine, phosphatidylethanolamine, phosphatidylserine, sphingolipid. Also included are water-soluble polymers, for example, gum Arabic, guar gum, carrageenan, agar, quince seed gum, locust bean gum, xanthan gum, pullulan, sodium carboxymethyl cellulose, hydroxyethyl cellulose, vinyl polymers such as carboxyvinyl polymer, and acrylic polymers such as (acryloyldimethyltaurine ammonium/vinyl pyrrolidone) copolymer, (sodium acrylate/sodium acryloyldimethyltaurine) copolymer, (hydroxyethyl acrylate/sodium acryloyldimethyltaurine) copolymer, (acrylamide/sodium acryloyldimethyltaurine) copolymer, and polyacrylamide. Using acrylic polymers, an O/W cosmetic composition can be stabilized in a relatively easy manner.

(3) Surfactant other than component (c)

The surfactant may be nonionic, anionic, cationic or ampholytic. The surfactant is not particularly limited as long as it is other than component (c) and commonly blended in cosmetics. Of the surfactants, one or more surfactants selected from non-crosslinked silicone surfactants and crosslinked silicone surfactants are preferred because a stable cosmetic composition is obtainable. In any cases, the amount of the surfactant blended is preferably 0.1 to 20% by weight of the overall cosmetic composition. An amount of at least 0.1% by weight is preferred in that the dispersing and emulsifying functions are fully achieved whereas an amount of up to 20% by weight is preferred because the risk that the cosmetic composition gives a sticky feeling on use is eliminated. The surfactant should preferably have a HLB of 2 to 14.5, though not limitative, for the purpose of maintaining the cosmetic composition water resistant.

The non-crosslinked silicone surfactants are straight or branched silicones in which some methyl groups on the backbone are substituted with hydrophilic groups such as polyethylene glycol and polyglycerin, for example, straight or branched polyoxyethylene-modified organopolysiloxanes, straight or branched polyoxyethylene/polyoxypropylene-modified organopolysiloxanes, straight or branched polyoxyethylene/alkyl-co-modified organopolysiloxanes, straight or branched polyoxyethylene/polyoxypropylene/alkyl-co-modified organopolysiloxanes, straight or branched polyglycerin-modified organopolysiloxanes, straight or branched polyglycerin/alkyl-co-modified organopolysiloxanes, and straight or branched pyrrolidone-modified organopolysiloxanes. Included are PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 polydimethylsiloxyethyl dimethicone (INCI), and lauryl polyglyceryl-3 polydimethylsiloxyethyl dimethicone (INCI).

Commercially available examples include KF-6011, KF-6011P, KF-6012, KF-6015, KF-6017, KF-6043, KF-6028, KF-6038, KF-6048, KF-6104, KF-6106, KF-6105, and KF-6115 by Shin-Etsu Chemical Co., Ltd.

Examples of the crosslinked silicone surfactant include (dimethicone/(PEG-10/15)) crosspolymer (INCI), (PEG-15/lauryl dimethicone) crosspolymer (INCI), (PEG-10/lauryl dimethicone) crosspolymer (INCI), (PEG-15/lauryl polydimethylsiloxyethyl dimethicone) crosspolymer (INCI), (dimethicone/polyglycerin-3) crosspolymer (INCI), (lauryl dimethicone/polyglycerin-3) crosspolymer (INCI), and (polyglycerin-3/lauryl polydimethylsiloxyethyl dimethicone) crosspolymer (INCI).

On use of a crosslinked silicone surfactant, it is preferred that in a composition consisting of the crosslinked silicone surfactant and an oil which is liquid at room temperature, the crosslinked silicone surfactant swell with the liquid oil as a result of being impregnated with an amount of more than its own weight of the liquid oil.

Suitable liquid oils include liquid silicone oils, hydrocarbon oils, ester oils, natural animal and plant oils, semi-synthetic oils and fluorochemical oils, all exemplified above as (1) oil. Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), and squalane (INCI).

Commercially available examples of the crosslinked silicone surfactant which is swollen with the liquid oil include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z from Shin-Etsu Chemical Co., Ltd.

(4) Powder other than component (a)

Suitable powders other than component (a) include coloring pigments, inorganic powders, metal powders, organic powders, and inorganic/organic composite powders, which are exemplified below.

• Coloring pigments

The coloring pigment is not particularly limited as long as it is commonly used in cosmetics for the purpose of coloring cosmetic compositions. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), cobalt titanate (labeling name, INCI: Cobalt Titanium Oxide), fired titanium/titanium oxide (labeling name, INCI: Titanium/Titanium Dioxide), Li/cobalt titanate (labeling name, INCI: Lithium Cobalt Titanate), cobalt titanate (labeling name, INCI: Cobalt Titanium Oxide), sintered iron oxide/titanium oxide (labeling name), composites doped with a different metal such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments such as γ-iron oxide, inorganic yellow pigments such as loess, colored pigments such as lake form of tar dyes and lake form of natural dyes. Any of the foregoing may be used.
The coloring pigment may take any shape including spherical, generally spherical, bar, spindle, petal, strip, and irregular shapes. Its geometry is not particularly limited as long as a color can be imparted to a cosmetic composition.

• Inorganic powder

Examples of the inorganic powder include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), talc (INCI), mica (INCI), kaolin (INCI), synthetic fluorphlogopite (labeling name, INCI: Synthetic Fluorphlogopite), synthetic gold mica iron (labeling name), black mica (labeling name, INCI: Biotite), potassium silicate (labeling name, INCI: Potassium Silicate), silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (INCI), bentonite (INCI), montmorillonite (INCI), hectorite (INCI), zeolite (INCI), alumina (INCI), Al hydroxide (labeling name, INCI: Aluminum Hydroxide), boron nitride (labeling name, INCI: Boron Nitride), and glass (labeling name, INCI: Glass).
Also, examples of the inorganic coloring pearly pigment include pearly agents such as mica (INCI) coated with titanium oxide (labeling name, INCI: Titanium dioxide), and synthetic fluorphlogopite (labeling name, INCI: Synthetic Fluorphlogopite) coated with titanium oxide (labeling name, INCI: Titanium Dioxide); and pearly pigments such as bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes (labeling name), and coloring mica coated with titanium oxide (labeling name, INCI: Titanium Dioxide), which may either be untreated or have undergone well-known surface treatment commonly used for cosmetics.

• Metal powder

Examples of the metal powder include metal microparticles such as aluminum (labeling name, INCI: Aluminum, Aluminum Powder), copper (labeling name, INCI: Copper Powder), silver (labeling name, INCI: Silver Powder), and gold (INCI: Gold).

• Organic powder

Examples of the organic powder include silicone, polyamide, polyacrylic acid-acrylate, polyester, polyethylene (INCI), polypropylene (INCI), polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, cellulose (INCI), silk (INCI), nylon (labeling name), phenolic resins, epoxy resins, polycarbonate, etc. in powder form.

In particular, as the silicone, silicone resin particles, e.g., polymethylsilsesquioxane (INCI), silicone rubber powder, and silicone resin-coated silicone rubber powder, (vinyl dimethicone/methicone silsesquioxane) crosspolymer (labeling name, INCI: Vinyl Dimethicone/Methicone Silsesquioxane Crosspolymer), (diphenyl dimethicone/vinyldiphenyl dimethicone/silsesquioxane) crosspolymer (labeling name, INCI: Diphenyl Dimethicone/Vinyl Diphenyl Dimethicone/-Silsesquioxane Crosspolymer), polysilicone-1 crosspolymer (INCI), and polysilicone-22 (INCI).

Commercially available examples of the silicone powder include KMP-590, KMP-591, KMP-592, KMP-597, KMP-598, KSP-100, KSP-101, KSP-102, KSP-105, KSP-300, KSP-411, KSP-441, KM-9729, and KM-440 from Shin-Etsu Chemical Co., Ltd.

Metal soaps are also included, examples of which include zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), K cetylphosphate (labeling name, INCI: Potassium Cetyl Phosphate) in powder form.

Also included are organic colorants, examples of which include tar dyes, specifically Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230 (1) (labeling name, INCI: Red 22, Red 22 Lake), Red #230 (2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 ((labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), Orange #207 (labeling name, INCI: Orange 11); and natural dyes, specifically cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

• Inorganic/organic composite powder

Exemplary of the inorganic/organic composite powder is a composite powder obtained by coating an inorganic powder on the surface with an organic powder by any well-known method.

The above-mentioned powders may be used as having been treated on the surface of particles. The surface treating agent capable of imparting hydrophobicity is preferred from the aspect of water resistance of a cosmetic composition. The hydrophobizing agent is not particularly limited. Exemplary treating agents include silicone treating agents, waxes, paraffins, organic fluorine compounds such as perfluoroalkyl phosphate salts, surfactants, amino acids such as N-acylglutamic acid, and metal soaps such as aluminum stearate and magnesium myristate.

Among others, the silicone treating agents are preferably used. Included are silanes or silylating agents such as triethoxycaprylylsilane (INCI); silicone oils such as dimethicone (INCI), methicone (INCI), hydrogendimethicone (INCI), triethoxysilylethyl polydimethylsiloxyethyl dimethicone (INCI), triethoxysilylethyl polydimethylsiloxyethylhexyl dimethicone (INCI), and acrylate/tridecyl acrylate/triethoxysilyl-propyl methacrylate/dimethicone methacrylate copolymers (labeling name, INCI: Acrylate/Tridecyl Acrylate/Triethoxysilylpropyl Methacrylate/Dimethicone Methacrylate Copolymer).

Examples of the silicone treating agent include AES-3083, KF-99P, KF-9901, KF-9908, KF-9909, KP-574, and KP-541, from Shin-Etsu Chemical Co., Ltd.

The surface hydrophobizing agent may be used alone or in admixture. Examples of the surface treated coloring pigment include KTP-09 series, especially KTP-09W, KTP-09R, KTP-09Y and KTP-09B from Shin-Etsu Chemical Co., Ltd.

• UV absorbing/scattering agent

In addition to the inventive dispersion, a dispersion having UV absorbing/scattering particles previously dispersed in an oil as component (a) may also be used.

Suitable oils include liquid silicone oils, hydrocarbon oils, ester oils, natural animal and plant oils, semi-synthetic oils and fluorochemical oils, all exemplified above as (1) oil.

Commercially available examples of the dispersion having UV absorbing/scattering particles previously dispersed in an oil include SPD series, specifically SPD-T5, SPD-Z5, SPD-T6, SPD-Z6, and SPD-T7, from Shin-Etsu Chemical Co., Ltd.

(5) Composition consisting of crosslinked organopolysiloxane and an oil which is liquid at room temperature

In the composition consisting of crosslinked organopolysiloxane and an oil which is liquid at room temperature, it is preferred that the crosslinked organopolysiloxane swell with the liquid oil as a result of being impregnated with an amount of more than its own weight of the liquid oil. Suitable liquid oils include liquid silicone oils, hydrocarbon oils, ester oils, natural animal and plant oils, semi-synthetic oils and fluorochemical oils, all exemplified above as (1) oil. Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), and squalane (INCI).

Unlike the crosslinked silicone surfactant as component (3) mentioned above, component (5) is a compound having neither polyether nor polyglycerin structure in its molecular structure. Examples include dimethicone/vinyl dimethicone crosspolymer (INCI), dimethicone/phenyl vinyl dimethicone crosspolymer (labeling name, INCI: Dimethicone/Phenyl Vinyl Dimethicone Crosspolymer), vinyl dimethicone/lauryl dimethicone crosspolymer (INCI), and lauryl polydimethylsiloxyethyl dimethicone/bisvinyl dimethicone crosspolymer (INCI).

Commercially available examples of the composition consisting of crosslinked organopolysiloxane and an oil which is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z from Shin-Etsu Chemical Co., Ltd.

(6) Film-forming agent

The film-forming agent is blended mainly for the purpose of further maintaining the effect-sustainability of the cosmetic composition. A silicone-based agent is preferred from the aspect of imparting water repellency though the agent is not limited thereto. Specifically use may be made of trimethylsiloxysilicic acid (labeling name, INCI: Trimethylsiloxysilicate), acrylic-silicone film formers, silicone-modified norbornene, silicone-modified pullulan, and silicone-modified polyvinyl alcohol.

Examples of the film-forming agent in the form of silicone-based agent herein include trimethylsiloxysilicic acid (labeling name, INCI: Trimethylsiloxysilicate), acrylate/dimethicone copolymer (INCI), norbornene/tris-(trimethylsiloxy) silylnorbornene copolymer (INCI), and pullulan tri(trimethylsiloxy)silylpropyl carbamide (labeling name, INCI: Trimethylsiloxysilylcarbamoyl Pullulan).

The film-forming agent may be previously dissolved in an oil which is liquid at room temperature before it is blended in the cosmetic composition. Suitable liquid oils used herein include liquid silicone oils, hydrocarbon oils, ester oils, natural animal and plant oils, semi-synthetic oils, and fluorochemical oils, all included in (1) oil as optional component.

Commercially available examples of the silicone film-forming agent include KF-7312J, KP-545, KP-549, KP-543, NBN-30-ID, TSPL-30-ID, and TSPL-30-D5 from Shin-Etsu Chemical Co., Ltd.

(7) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

• Oil-soluble gelling agent

Exemplary oil-soluble gelling agents include metal soaps such as aluminum stearate, magnesium stearate and zinc myristate; amino acid derivatives such as lauroyl glutamic acid (labeling name, INCI: Lauroyl Glutamic Acid ) and $\alpha,\gamma$-di-n-butylamine; dextrin fatty acid esters such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), dextrin palmitate/ethylhexanoate (labeling name, INCI: Dextrin Palmitate/Ethylhexanoate); sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; organo-modified clay minerals such as disteardimonium hectorite (INCI), stearalkonium hectorite (INCI), and hectorite; and stearalkonium bentonite (INCI).

• Preservative/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, bisabolol, ethyl hexyl glycerin, glyceryl caprylate, caprylhydroxaminc acid, hexyl dimethylpropanoate, polyaminopropyl biguanide, photosensitizers, silver, and plant extracts.

• Antiperspirant

Examples of the antiperspirant include aluminum halohydrates such as aluminum chlorohydrate, aluminum halides such as aluminum chloride, aluminum allantoinate, tannic acid, persimmon, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components which exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine.

• Fragrance

Fragrances include natural fragrances and synthetic fragrances. Suitable natural fragrances include plant fragrances separated from flowers, leaves, stems, and pericarps, and animal fragrances such as musk and civet. Suitable synthetic fragrances include hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

• Salt

Salts include inorganic salts, organic acid salts, amine salts, and amino acid salts. Exemplary inorganic salts include sodium, potassium, magnesium, calcium, aluminum, zirconium and zinc salts of inorganic acids such as hydrochloric acid, sulfuric acid, carbonic acid and nitric acid. Exemplary organic acid salts include salts of organic acids such as acetic acid, dehydroacetic acid, citric acid, malic acid, succinic acid, ascorbic acid, and stearic acid. Exemplary amine salts and amino acid salts include salts of amines such as triethanol amine, and salts of amino acids such as glutamic acid. Also useful are hyaluronic acid, salts such as chondroitin sulfate, aluminum zirconium glycine complex, and acid-alkali neutralized salts used in cosmetic formulations. In particular, sodium chloride is preferred from the aspects of dissolution, feeling on use, and anti-foaming. When used in large amounts, the salt can inhibit the water-soluble polymer from swelling.

• Antioxidant

Examples of the antioxidant include, but are not particularly limited to, carotenoid, ascorbic acid and salts thereof, ascorbyl stearate, tocopherol, tocopherol acetate, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used alone or in admixture.

• Acidity regulator

Suitable acidity regulators include lactic acid, citric acid, glycolic acid, succinic acid, tartaric acid, dl-malic acid, potassium carbonate, sodium hydrogencarbonate, and ammonium hydrogencarbonate.

• Chelating agent

Suitable chelating agents include alanine, sodium EDTA, sodium polyphosphate, sodium metaphosphate, and phosphoric acid.

• Refreshing agent

Suitable refreshing agents include L-menthol, camphor and menthyl lactate.

• Anti-inflammatory agent

Suitable anti-inflammatory agents include allantoin, glycyrrhizinic acid and salts thereof, glycyrrhetinic acid, stearyl glycyrrhetinate, tranexamic acid, and azulene.

• Skin modifying ingredient

Suitable skin modifying ingredients include brightening or whitening agents such as placenta extract, arbutin, glutathione, and Saxifraga Sarmentosa Extract; cell activators such as royal jelly, photosensitizers, cholesterol derivatives, and bovine blood extracts; anti-skin-roughening agents; blood flow enhancing agents such as vanillylamide nonanoate, benzyl nicotinate, $\beta$-butoxyethyl nicotinate, capsaicin, zingerone, cantharides tincture, ichthammol, caffeine, tannic acid, $\alpha$-borneol, tocopherol nicotinate, inositol hexanicotinate, cyclandelate, cinnarizine, tolazoline, acetylcholine, verapamil, cepharanthine, and $\gamma$-oryzanol; skin astringents such as zinc oxide and tannic acid; and antiseborrheic agents such as sulfur and thianthol.

• Vitamin

Suitable vitamins include vitamin A family such as vitamin A oil, retinol, retinol acetate, retinol palmitate; vitamin B family, specifically vitamin B2 family such as riboflavin, riboflavin butyrate, and flavin adenine nucleotide, vitamin B6 family such as pyridoxine hydrochloride, pyridoxine dioctanoate, pyridoxine tripalmitate, vitamin B12 and derivatives thereof, vitamin B15 and derivatives thereof; vitamin C family such as L-ascorbic acid, L-ascorbic acid dipalmitic acid ester, L-ascorbic acid-2-sodium sulfate, and dipotassium L-ascorbic acid phosphoric acid diester; vitamin D family such as ergocalciferol and cholecalciferol; vitamin E family such as $\alpha$-tocopherol, $\beta$-tocopherol, $\gamma$-tocopherol, dl-$\alpha$-tocopherol acetate, dl-$\alpha$-tocopherol nicotinate, and dl-$\alpha$-tocopherol succinate; nicotinic acids such as nicotinic acid, benzyl nicotinate, and nicotinic amide; vitamin H, vitamin P, pantothenic acids such as calcium pantothenate, D-pantothenyl alcohol, pantothenyl ethyl ether, acetylpantothenyl ethyl ether; and biotin.

• Amino acid

Suitable amino acids include glycine, valine, leucine, isoleucine, serine, threonine, phenylalanine, arginine, lysine, aspartic acid, glutamic acid, cystine, cysteine, methionine, and tryptophan.

• Nucleic acid

Typical of the nucleic acid is deoxyribonucleic acid.

• Hormone

Suitable hormones include estradiol and ethenyl estradiol.

• Clathrate compound

Typical of the clathrate compound is cyclodextrin.

EXAMPLES

Examples and Comparative Examples are shown below by way of illustration and not by way of limitation. In Examples, compositional percent (%) and ratio are by weight unless otherwise stated. The amount of product name is the amount of the product blended.

[Example of dispersion]

A dispersion was obtained by preparing a slurry on a roll mill in accordance with the formulation shown in Table 1. The dispersion was evaluated for dispersibility according to the criteria shown below. The results are also shown in Table 1.

[Dispersibility in water]

Dispersibility was evaluated by taking a 0.5 g sample of the dispersion with a metal spatula, putting the sample in a beaker filled with 50 mL of water, and stirring.

◎: uniformly dispersed, with no sedimentation
○: temporarily sedimented, but uniformly dispersed as stirring continued
×: not dispersed

The sample was rated with "○" for good or above were judged "Pass".

[Table 1]

| Formulation (%) | Example | | | | Comparative Example | | | | |
|---|---|---|---|---|---|---|---|---|---|
| | 1 | 2 | 3 | 4 | 1 | 2 | 3 | 4 | 5 |
| (a) MT-200ST (*1) | 60 | 70 | 60 | - | 60 | 60 | 80 | 60 | 60 |
| (a) MT-150EX (*2) | - | - | - | 60 | - | - | - | - | |
| (b) BG | 25 | 15 | 20 | 25 | 40 | - | 10 | 33 | 18 |
| (c) KF-6100 (*3) | 15 | 15 | 20 | 15 | - | 40 | 10 | 7 | 22 |
| Total | 100 | 100 | 100 | 100 | 100 | 100 | 100 | 100 | 100 |
| (c)/(b) | 0.6 | 1.0 | 1.0 | 0.6 | 0 | - | 1.0 | 0.21 | 1.22 |
| Dispersibility in water | ◎ | ○ | ○ | ○ | × | × | × | × | × |

(*1) fine particle of titanium oxide (labeling name, INCI: Titanium Dioxide) treated with stearic acid (labeling name, INCI: Stearic Acid), having a number average primary particle diameter of 20 nm, as determined by image analysis of a transmission electron photomicrograph
(*2) fine particle of titanium oxide (labeling name, INCI: Titanium Dioxide) treated with isostearic acid (labeling name, INCI: Isostearic Acid) and aluminum hydroxide (labeling name, INCI: Aluminum Hydroxide), having a number average primary particle diameter of 15 nm, as determined by image analysis of a transmission electron photomicrograph
(*3) polyglyceryl-3 disiloxane dimethicone (labeling name, INCI: Polyglyceryl-3 Disiloxane Dimethicone), soluble in component (b)

As is evident from Table 1, dispersions (or slurries) having excellent dispersibility in water were obtained from all Examples within the scope of the invention. On the other hand, Comparative Example 1 not containing component (c), Comparative Example 2 not containing component (b), Comparative Example 3 containing at least 70% of component (a), Comparative Example 4 containing at least 30% of component (b), and Comparative Example 5 containing at least 20% of component (c) showed inferior dispersibility in water.
Slurries were prepared according to the formulation shown in Tables 2 to 5 using a roll mill. Whether or not a dispersion could be prepared was evaluated according to the criteria shown below. The results are also shown in Tables 2 to 5.

[Whether or not a dispersion could be prepared]

O: a dispersion is obtainable
×: a dispersion is not obtainable

The sample was rated with "○" was judged "Pass".

[Evaluation of viscosity]

The dispersion was evaluated for viscosity according to the criteria shown below. The results of Examples 5 to 19 and Comparative Examples 6 to 25 are shown in Tables 2 to 5. Viscosity was measured at 25°C according to the method prescribed in JIS K7117-1: 1999 using a 50-mL vial and a Brookfield viscometer (TVB-10 type, Toki Sangyo Co., Ltd.).

[Evaluation of viscosity stability (or dispersion stability)]

The dispersion was evaluated for stability according to the criteria shown below. The results are also shown in Tables 2 to 5.

The viscosity of a dispersion immediately after preparation and a dispersion which was stored at room temperature for 2 weeks was measured at 25°C according to the method prescribed in JIS K7117-1: 1999 using a 50-mL vial and a Brookfield viscometer (TVB-10 type, Toki Sangyo Co., Ltd.). A percent change of the viscosity (mPa·s) after 2 weeks from the initial viscosity (mPa·s) was computed according to the following formula.

(Viscosity change) =

$$[100 \times \{(\text{viscosity after 2 weeks (mPa·s)}) - (\text{initial viscosity (mPa·s)})\} / (\text{initial viscosity (mPa·s)}) ]$$

O: viscosity change < 30%
△: 30% ≤ viscosity change < 50%
✕: 50% ≤ viscosity change

The sample was rated with "△" for fair or above were judged "Pass".

[Table 2]

| Formulation (%) | Example | | | | | | |
|---|---|---|---|---|---|---|---|
| | 5 | 6 | 7 | 8 | 9 | 10 | 11 |
| (a) MT-200ST (*1) | 50 | 50 | 30 | 20 | 10 | 20 | 20 |
| (b) BG | 10 | 10 | 7 | 5 | 5 | 1.5 | 1 |
| (c) KF-6100 (*3) | 7 | 8 | 4 | 3 | 3 | 1 | 1 |
| (d) Water | 33 | 32 | 59 | 72 | 82 | 77.5 | 78 |
| Total | 100 | 100 | 100 | 100 | 100 | 100 | 100 |
| (c)/(b) | 0.7 | 0.8 | 0.57 | 0.6 | 0.60 | 0.67 | 1.0 |
| Possibility of preparation | O | ○ | ○ | ○ | ○ | ○ | ○ |
| Viscosity (mPa·s) | 318 | 818 | 184 | 128 | 28 | 753 | 1,120 |
| Viscosity stability | ○ | ○ | ○ | ○ | Δ | Δ | Δ |

[Table 3]

| Formulation (%) | Example | | | | | | | |
|---|---|---|---|---|---|---|---|---|
| | 12 | 13 | 14 | 15 | 16 | 17 | 18 | 19 |
| (a) MT-200ST (*1) | 50 | - | 50 | 50 | 50 | 50 | 50 | 50 |
| (a) MT-100TV (*2) | - | 40 | - | - | - | - | - | - |
| (b) BG | 14.5 | 10 | 30 | 8 | 15 | - | - | 10 |
| (b) DPG | - | - | - | - | - | 10 | - | - |
| (b) Glycerin | - | - | - | - | - | - | 10 | - |
| (c) KF-6100 (*3) | 5.1 | 9 | 12 | 7 | 7 | 7 | 7 | 10 |
| (d) Water | 30.4 | 41 | 8 | 35 | 28 | 33 | 33 | 30 |

(continued)

| Formulation (%) | Example | | | | | | | |
|---|---|---|---|---|---|---|---|---|
| | 12 | 13 | 14 | 15 | 16 | 17 | 18 | 19 |
| Total | 100 | 100 | 100 | 100 | 100 | 100 | 100 | 100 |
| (c)/(b) | 0.35 | 0.9 | 0.4 | 0.88 | 0.47 | 0.7 | 0.7 | 1.0 |
| Possibility of preparation | ○ | ○ | ○ | ○ | ○ | ○ | ○ | ○ |
| Viscosity (mPa·s) | 1,321 | 63 | 1,030 | 259 | 147 | 94 | 2,820 | 4,800 |
| Viscosity stability | Δ | Δ | Δ | ○ | ○ | Δ | Δ | Δ |

[Table 4]

| Formulation (%) | Comparative Example | | | | | | | | |
|---|---|---|---|---|---|---|---|---|---|
| | 6 | 7 | 8 | 9 | 10 | 11 | 12 | 13 | 14 |
| (a) MT-200ST (*1) | 50 | 50 | 50 | 50 | 50 | 50 | 50 | 50 | 50 |
| (b) BG | 10 | 10 | 10 | 10 | 10 | 10 | 10 | 10 | 13 |
| KF-6013 (*5) | 7 | - | - | - | - | - | - | - | 12 |
| KF-6104 (*6) | - | 7 | - | - | - | - | - | - | - |
| KF-6105 (*7) | - | - | 7 | - | - | - | - | - | - |
| KF-6106 (*8) | - | - | - | 7 | - | - | - | - | - |
| KF-6017 (*9) | - | - | - | - | 7 | - | - | - | - |
| KF-6011 (*10) | - | - | - | - | - | 7 | - | - | - |
| KF-6043 (*11) | - | - | - | - | - | - | 7 | - | - |
| KP-578 (*12) | - | - | - | - | - | - | - | 7 | - |
| (d) Water | 33 | 33 | 33 | 33 | 33 | 33 | 33 | 33 | 25 |
| Total | 100 | 100 | 100 | 100 | 100 | 100 | 100 | 100 | 100 |
| (c)/(b) | 0.7 | 0.7 | 0.7 | 0.7 | 0.7 | 0.7 | 0.7 | 0.7 | 0.9 |
| Possibility of preparation | × | × | × | × | × | × | × | × | ○ |
| Viscosity (mPa·s) | - | - | - | - | - | - | - | - | 7,630 |
| Viscosity stability | - | - | - | - | - | - | - | - | × |

[Table 5]

| Formulation (%) | Comparative Example | | | | | | | | | | |
|---|---|---|---|---|---|---|---|---|---|---|---|
| | 15 | 16 | 17 | 18 | 19 | 20 | 21 | 22 | 23 | 24 | 25 |
| (a) MT-200ST (*1) | - | - | - | - | - | - | - | - | - | 50 | 50 |
| (a) MT-100TV (*2) | - | 40 | 40 | 40 | 40 | 40 | 40 | 40 | 40 | - | - |
| MT-100WP (*4) (comparative component) | 30 | - | - | - | - | - | - | - | - | - | - |
| (b) BG | 10 | 10 | 10 | 10 | 10 | 10 | 10 | 10 | 10 | 8 | 0.5 |
| (c) KF-6100 (*3) | 5 | - | - | - | - | - | - | - | - | 0.5 | 7 |
| KF-6013 (*5) | - | 10 | - | - | - | - | - | - | - | - | - |
| KF-6104 (*6) | - | - | 10 | - | - | - | - | - | - | - | - |
| KF-6105 (*7) | - | - | - | 10 | - | - | - | - | - | - | - |
| KF-6106 (*8) | - | - | - | - | 10 | - | - | - | - | - | - |
| KF-6017 (*9) | - | - | - | - | - | 10 | - | - | - | - | - |

(continued)

| Formulation (%) | Comparative Example | | | | | | | | | | |
|---|---|---|---|---|---|---|---|---|---|---|---|
| | 15 | 16 | 17 | 18 | 19 | 20 | 21 | 22 | 23 | 24 | 25 |
| KF-6011 (*10) | - | - | - | - | - | - | 10 | - | - | - | - |
| KF-6043 (*11) | - | - | - | - | - | - | - | 10 | - | - | - |
| KP-578 (*12) | - | - | - | - | - | - | - | - | 10 | - | - |
| (d) Water | 55 | 40 | 40 | 40 | 40 | 40 | 40 | 40 | 40 | 41.5 | 42.5 |
| Total | 100 | 100 | 100 | 100 | 100 | 100 | 100 | 100 | 100 | 100 | 100 |
| (c)/(b) | 0.5 | 1.0 | 1.0 | 1.0 | 1.0 | 1.0 | 1.0 | 1.0 | 1.0 | 0.06 | 14.0 |
| Possibility of preparation | ○ | × | × | × | × | × | × | × | × | × | × |
| Viscosity (mPa·s) | 820 | - | - | - | - | - | - | - | - | - | - |
| Viscosity stability | × | - | - | - | - | - | - | - | - | - | - |

(*1) fine particle of titanium oxide (labeling name, INCI: Titanium Dioxide) treated with stearic acid (labeling name, INCI: Stearic Acid) (*2) fine particle of titanium oxide (labeling name, INCI: Titanium Dioxide) treated with stearic acid (labeling name, INCI: Stearic Acid) and aluminum hydroxide (labeling name, INCI: Aluminum Hydroxide), having a number average primary particle diameter of 15 nm, as determined by image analysis of a transmission electron photomicrograph (*3) polyglyceryl-3 disiloxane dimethicone (labeling name, INCI: Polyglyceryl-3 Disiloxane Dimethicone) (*4) comparative component, fine particle of titanium oxide (labeling name, INCI: Titanium Dioxide) treated with hydrous silica (labeling name, INCI: Hydrated Silica), having a number average primary particle diameter of 15 nm, as determined by image analysis of a transmission electron photomicrograph (*5) PEG-9 dimethicone (labeling name, INCI: PEG-9 Dimethicone) (*6) polyglyceryl-3 polydimethylsiloxyethyl dimethicone (labeling name, INCI: Polyglyceryl-3 Polydimethylsiloxyethyl Dimethicone), insoluble in component (b) (*7) lauryl polyglyceryl-3 polydimethylsiloxyethyl dimethicone (labeling name, INCI: Lauryl Polyglyceryl-3 Polydimethylsiloxyethyl Dimethicone), insoluble in component (b) (*8) polyglyceryl-3 polydimethylsiloxyethyl dimethicone (labeling name, INCI: Polyglyceryl-3 Polydimethylsiloxyethyl Dimethicone), insoluble in component (b) (*9) PEG-10 dimethicone (labeling name, INCI: PEG-10 Dimethicone) (*10) PEG-11 methyl ether dimethicone (labeling name, INCI: PEG-11 Methyl Ether Dimethicone) (*11) PEG-10 dimethicone (labeling name, INCI: PEG-10 Dimethicone) (*12) acrylates/ethylhexyl acrylate/dimethicone methacrylate copolymer (labeling name, INCI: Acrylates/Ethylhexyl Acrylate/Dimethicone Methacrylate Copolymer), insoluble in component (b)

As is evident from Tables 2 to 5, all the dispersions obtained in Examples 5 to 19 had a low viscosity. In Comparative Examples 6 to 25, dispersions could not be prepared, or when dispersions could be prepared, they had a high viscosity or poor viscosity stability.

[Example 20 and Comparative Examples 26 to 27]

An O/W cream was prepared according to the formulation shown in Table 6.

[Preparation method]

A: Mixing component (2).
B: Mixing component (3).
C: Adding the mixture of A to the mixture of B and emulsifying.
D: Adding component (1) to the emulsion of C and mixing to prepare an O/W type cream.

[Evaluation of transparency and feeling on use]

The cosmetic composition was evaluated for transparency on application and feeling on use (or free of stickiness) by a panel of 10 professional members. The result is an average of ratings of 10 panel members and rated according to the following judgment criterion.

[Evaluation criterion]

5 points:     excellent
4 points:     good
3 points:     mediocre
2 points:     rather poor
1 point:      poor

After the average point was computed, the sample was rated according to the following criterion.

[Judgment criterion]

◎: 4.5 ≤ average point
○: 3.5 ≤ average point < 4.5
△: 2.5 ≤ average point < 3.5
✕: average point < 2.5

The sample was rated with "△" for fair or above were judged "Pass".

[Water resistance evaluation]

The cosmetic composition was evaluated for water resistance on application according to the following criterion. The result was judged according to the following criterion.

[Judgment criterion]

○: remain after 1 minute flow of water
✕: vanish after 1 minute flow of water

The sample was rated with "○" was judged "Pass".

[Table 6]

| Formulation (%) | | Example | Comparative Example | |
|---|---|---|---|---|
| | | 20 | 26 | 27 |
| (1) | Dispersion of Example 5 (50%) | 18 | - | - |
| | Dispersion of Comparative Example 14 (50%) | - | 18 | - |
| | Dispersion of Comparative Example 15 (30%) | - | - | 30 |

(continued)

| Formulation (%) | | | Example | Comparative Example | |
|---|---|---|---|---|---|
| | | | 20 | 26 | 27 |
| (2) | Dimethicone | | 3 | 3 | 3 |
| | (Ammonium acryloyl dimethyltaurine/VP) copolymer | | 0.2 | 0.2 | 0.2 |
| | (Sodium acrylate/sodium acryloyl dimethyltaurine) copolymer | | 2 | 2 | 2 |
| | Polyglyceryl-10 myristate | | 0.2 | 0.2 | 0.2 |
| | Polyglyceryl-10 diisostearate | | 0.8 | 0.8 | 0.8 |
| (3) | BG | | 6 | 6 | 6 |
| | Alcohol | | 6 | 6 | 6 |
| | Phenoxyethanol | | 0.2 | 0.2 | 0.2 |
| | Water | | 63.6 | 63.6 | 51.6 |
| Total | | | 100.0 | 100.0 | 100.0 |
| Transparency | | | ◎ | ○ | × |
| Feeling on use | | | ◎ | × | × |
| Water resistance | | | ○ | ○ | × |

As is evident from Table 6, the cosmetic composition of Example 20 using the dispersion within the scope of the invention showed high transparency on application, a non-sticky pleasant feeling on use, and water resistance.

[Example 21] O/W sunscreen

| | Formulation | % |
|---|---|---|
| 1. | KSG-15 (*1) | 8.0 |
| 2. | KSG-16 (*2) | 24.0 |
| 3. | Cyclopentasiloxane | 10.0 |
| 4. | BG | 3.0 |
| 5. | KF-6100 (*3) | 0.6 |
| 6. | KF-6104 (*4) | 0.3 |
| 7. | (Ammonium acryloyl dimethyltaurine/VP) copolymer aqueous solution (*5) | 13.0 |
| 8. | (Acrylamide/sodium acryloyl dimethyltaurine) copolymer/ isohexadecane/polysorbate 80 aqueous solution (*6) | 0.6 |
| 9. | 1% aqueous solution of sodium chloride | 8.0 |
| 10. | Water | 12.5 |
| 11. | Silicone-treated fine particle of titanium oxide (*7) 5%/ BG 1%/KF-6100 0.7%/water 3.3% | 10.0 |
| 12. | Silicone-treated fine particle of zinc oxide (*8) 5%/ BG 1%/KF-6100 0.7%/water 3.3% | 10.0 |

(continued)

| Formulation | % |
|---|---|
| Total | 100.0 |

(*1) mixture of cyclopentasiloxane 90-96% + (dimethicone/vinyl dimethicone) crosspolymer 4-10%, Shin-Etsu Chemical Co., Ltd.

(*2) mixture of dimethicone 70-80% + (dimethicone/vinyl dimethicone) crosspolymer 20-30%, Shin-Etsu Chemical Co., Ltd.

(*3) polyglyceryl-3 disiloxane dimethicone, Shin-Etsu Chemical Co., Ltd.

(*4) polyglyceryl-3 polydimethylsiloxyethyl dimethicone, Shin-Etsu Chemical Co., Ltd.

(*5) Aristoflex AVC, Clariant AG (*6) Simulgel 600, Seppic (*7) treated with KF-9901 (hydrogen dimethicone), Shin-Etsu Chemical Co., Ltd.

(*8) treated with AES-3083 (triethoxycaprylyl-silane), Shin-Etsu Chemical Co., Ltd.

(Preparation method)

A: Uniformly mixing ingredients 1 to 3.
B: Uniformly mixing ingredients 4 to 10.
C: Uniformly dispersing ingredients 11 and 12 on a bead mill.
D: Adding A to B, emulsifying, adding C to the emulsion, and uniformly mixing.

The O/W sunscreen thus obtained showed high stability, high transparency, a non-sticky pleasant feeling on use, and water resistance. Ingredients 11 and 12 were highly stable and easy to handle and admit.

[Example 22] Aqueous gel

| | Formulation | % |
|---|---|---|
| 1. | Dispersion of Example 1 | 5.0 |
| 2. | Ethanol | 3.5 |
| 3. | BG | 4.0 |
| 4. | Glycerin | 2.0 |
| 5. | (Ammonium acryloyl dimethyltaurine/VP) copolymer | 0.2 |
| 6. | Xanthan gum | 0.2 |
| 7. | Arginine | 0.5 |
| 8. | Phenoxyethanol | 0.3 |
| 9. | Water | balance |
| | Total | 100.0 |

(Preparation method)

A: Uniformly mixing ingredients 1 and 2.
B: Uniformly mixing ingredients 3 to 9.
C: Adding A to B and uniformly mixing them.
D: Debubbling C and filling a container therewith to prepare an aqueous gel.

The aqueous gel thus obtained showed full freshness on application, high stability, high transparency, a non-sticky pleasant feeling on use, and water resistance. Ingredient 1 was highly stable and easy to handle and admit.

[Example 23] O/W base cream

| | Formulation | % |
|---|---|---|
| 1. | Water | balance |
| 2. | Glycerin | 3.0 |
| 3. | Microcrystalline wax | 3.0 |
| 4. | Xanthan gum | 0.2 |
| 5. | Pentylene glycol | 2.0 |
| 6. | BG | 5.0 |
| 7. | Stearic acid-treated fine particle of titanium oxide | 5.0 |
| 8. | BG | 1.0 |
| 9. | KF-6106 (*1) | 0.1 |
| 10. | KF-6100 (*2) | 0.7 |
| 11. | Water | 3.3 |
| 12. | Sucrose cocoate | 0.2 |
| 13. | Sorbitan stearate | 3.0 |
| 14. | PEG-60 glyceryl isostearate | 0.5 |
| 15. | Behenyl alcohol | 0.5 |
| 16. | Ethylhexyl palmitate | 3.0 |
| 17. | KSP-101 (*3) | 3.0 |
| 18. | Triethylhexanoin | 6.0 |
| 19. | Polyhydroxystearic acid | 0.5 |
| 20. | KTP-09W (*4) | proper |
| 21. | KTP-09R (*4) | proper |
| 22. | KTP-09Y (*4) | proper |
| 23. | KTP-09B (*4) | proper |
| 24. | Polysorbate 60 | 0.3 |
| 25. | (Hydroxyethyl acrylate/sodium acryloyldimethyltaurine) copolymer (*5) | 0.6 |
| | Total | 100.0 |

(*1) polyglyceryl-3 polydimethylsiloxyethyl dimethicone, Shin-Etsu Chemical Co., Ltd.
(*2) polyglyceryl-3 disiloxane dimethicone, Shin-Etsu Chemical Co., Ltd.
(*3) (vinyl dimethicone/methicone silsesquioxane) crosspolymer, Shin-Etsu Chemical Co., Ltd.
(*4) coloring inorganic pigments treated with KF-9909, W: white, R: red, Y: yellow, B: black, Shin-Etsu Chemical Co., Ltd.
(*5) Simulgel EG, Seppic

(Preparation method)

A: Uniformly dispersing ingredients 7 to 11 on a bead mill.
B: Uniformly mixing ingredients 1 to 6 and mixing with A until uniform.
C: Heating and dissolving ingredients 12 to 16 and mixing with ingredient 17 until uniform.
D: Mixing ingredients 18 to 23 and milling on a roll mill.
E: Adding the mixture of D to the mixture of C and uniformly mixing.
F: Adding the heated mixture of E to the heated mixture of B and uniformly emulsifying.
G: Cooling the mixture of F to room temperature, adding ingredients 23 and 24, and uniformly mixing.
H: Debubbling the mixture of G and filling a container therewith to prepare an O/W base cream.

The O/W base cream thus obtained showed high stability, high transparency, a non-sticky pleasant feeling on use, and water resistance. The component of A in the preparation method was highly stable and easy to handle and admit.

[Example 24] O/W sunscreen milky lotion

| | Formulation | % |
|---|---|---|
| 1. | MT-200ST (*1) | 7.5 |
| 2. | Ethanol | 0.5 |
| 3. | BG | 1.0 |
| 4. | Water | 5.0 |
| 5. | KF-6100 (*2) | 1.2 |
| 6. | Ethanol | 9.5 |
| 7. | BG | 5.0 |
| 8. | Methyl paraben | 0.1 |
| 9. | Sodium acrylate/sodium acryloyldimethyltaurate copolymer (*3) | 2.5 |
| 10. | Water | balance |
| 11. | KF-7312J (*4) | 1.0 |
| 12. | KF-56A (*5) | 3.0 |
| 13. | KSG-016F (*6) | 1.0 |
| 14. | Cetyl alcohol | 2.0 |
| 15. | Ethyl hexyl methoxycinnamate | 5.0 |
| 16. | Hexyl diethylaminohydroxybenzoylbenzoate | 1.0 |
| 17. | Polyoxyethylene (60) hydrogenated castor oil | 1.0 |
| 18. | KF-6011 (*7) | 0.5 |
| 19. | Tocopherol | 0.05 |
| | Total | 100.0 |

(*1) stearic acid-treated fine particle of titanium oxide
(*2) polyglyceryl-3 disiloxane dimethicone, Shin-Etsu Chemical Co., Ltd.
(*3) Simulgel EG, Seppic
(*4) 0% trimethylsiloxysilicic acid in cyclopentasiloxane, Shin-Etsu Chemical Co., Ltd.
(*5) diphenylsiloxyphenyl trimethicone, Shin-Etsu Chemical Co., Ltd.
(*6) mixture of dimethicone 70-80% + (dimethicone/vinyl dimethicone) crosspolymer 20-30%, Shin-Etsu Chemical Co., Ltd.
(*7) PEG-11 methyl ether dimethicone, Shin-Etsu Chemical Co., Ltd.

(Preparation method)

A: Uniformly dispersing ingredients 1 to 5 on a bead mill.
B: Heating ingredients 6 to 10 at 85°C, adding A thereto, and uniformly mixing.
C: Heating ingredients 11 to 19 at 85°C and uniformly mixing.
D: Adding C to B, emulsifying at 85°C, and slowly cooling with stirring to prepare an O/W sunscreen milky lotion.

The O/W sunscreen milky lotion thus obtained showed high stability, high transparency, a non-sticky pleasant feeling on use, and water resistance. The component of A in the preparation method was highly stable and easy to handle and admit.

[Example 25] O/W primer

| | Formulation | % |
|---|---|---|
| 1. | KP-545 (*1) | 3 |

(continued)

| | Formulation | % |
|---|---|---|
| 2. | KSG-19 (*2) | 5 |
| 3. | KF-56A (*3) | 5 |
| 4. | Cyclopentasiloxane | 10 |
| 5. | MT-100TV (*4) | 7.2 |
| 6. | BG | 1.8 |
| 7. | KF-6100 (*5) | 1.6 |
| 8. | Water | 20 |
| 9. | BG | 10 |
| 10. | Betaine | 1 |
| 11. | KF-6043 (*6) | 1.5 |
| 12. | Sodium acrylate-sodium acryloyldimethyltaurate copolymer (*7) | 1 |
| 13. | (acrylate/alkyl (C10-30) acrylate) crosscopolymer (2% aqueous solution) | 20 |
| 14. | Arginine (10% aqueous solution) | proper |
| 15. | Bisabolol | 0.1 |
| 16. | Ethylhexylglycerin | 0.1 |
| 17. | EDTA-2Na (10% aqueous solution) | 0.1 |
| 18. | Water | balance |
| | Total | 100.0 |

(*1) solution of 30% (acrylate/dimethicone) copolymer in cyclopentasiloxane, Shin-Etsu Chemical Co., Ltd.

(*2) mixture of dimethicone 80-90% + (dimethicone/vinyl dimethicone) crosspolymer 10-20%, Shin-Etsu Chemical Co., Ltd.

(*3) diphenylsiloxyphenyl trimethicone, Shin-Etsu Chemical Co., Ltd.

(*4) fine particle of titanium oxide treated with stearic acid/aluminum hydroxide (*5) polyglyceryl-3 disiloxane dimethicone, Shin-Etsu Chemical Co., Ltd.

(*6) PEG-10 dimethicone, Shin-Etsu Chemical Co., Ltd.

(*7) Simulgel EG, Seppic

(Preparation method)

A: Uniformly mixing ingredients 1 to 4.
B: Uniformly dispersing ingredients 5 to 8 on a homo-mixer.
C: Uniformly mixing ingredients 9 to 18 and B.
D: Adding B to C and emulsifying to prepare an O/W primer.

The O/W primer thus obtained showed high stability, high transparency, a non-sticky pleasant feeling on use, and water resistance. The dispersion of A in the preparation method was highly stable and easy to handle and admit.

[Example 26] O/W liquid foundation

| | Formulation | % |
|---|---|---|
| 1. | Stearic acid | 1.0 |
| 2. | Behenyl alcohol | 0.4 |
| 3. | Glyceryl stearate | 0.3 |
| 4. | Mineral oil | 10.0 |
| 5. | Glyceryl trioctanoate | 5.0 |
| 6. | KP-561P (*1) | 3.0 |
| 7. | Sorbitan sesquioleate | 0.5 |

(continued)

| | Formulation | % |
|---|---|---|
| 8. | Sorbitan monooleate | 1.0 |
| 9. | Acrylate copolymer | 2.2 |
| 10. | Triethanol amine | 1.0 |
| 11. | KF-6013 (*2) | 0.2 |
| 12. | Alkyl-POE palmityl ether phosphate | 0.1 |
| 13. | POE hydrogenated castor oil | 0.5 |
| 14. | Silicone-treated titanium oxide (*3) | 8.5 |
| 15. | Silicone-treated red iron oxide (*3) | 0.4 |
| 16. | Silicone-treated yellow iron oxide (*3) | 1.0 |
| 17. | Silicone-treated black iron oxide (*3) | 0.1 |
| 18. | MT-200ST (*4) | 5.0 |
| 19. | Ethanol | 3.0 |
| 20. | KF-6100 (*5) | 0.7 |
| 21. | Water | 3.3 |
| 22. | BG | 7.0 |
| 23. | Preservative | proper |
| 24. | Fragrance | proper |
| 25. | Water | balance |
| | Total | 100.0 |

(*1) (acrylate/stearyl acrylate/dimethicone methacrylate) copolymer, Shin-Etsu Chemical Co., Ltd.
(*2) PEG-9 dimethicone, Shin-Etsu Chemical Co., Ltd.
(*3) triethoxycaprylylsilane-treated pigments, Shin-Etsu Chemical Co., Ltd.
(*4) stearic acid-treated fine particle of titanium oxide
(*5) polyglyceryl-3 disiloxane dimethicone, Shin-Etsu Chemical Co., Ltd.

(Preparation method)

A: Mixing ingredients 11 to 13 with a portion of 22, adding ingredients 14 to 17, uniformly dispersing, and heating.
B: Mixing ingredients 1 to 8 and heating for dissolution.
C: Mixing ingredients 9 to 10, the remainder of 22, 23 and 25 and heating.
D: Dispersing ingredients 18 to 21 on a dispersing mixer.
E: Stirring, adding B to C, emulsifying, adding A, and further adding D and ingredient 24 to prepare an O/W liquid foundation.

The O/W liquid foundation thus obtained showed high stability, high transparency, a non-sticky pleasant feeling on use, and water resistance. The dispersion of D in the preparation method was highly stable and easy to handle and admit.

[Example 27] W/O sunscreen milky lotion

| | Formulation | % |
|---|---|---|
| 1. | KSG-210 (*1) | 3.0 |
| 2. | KSG-15 (*2) | 2.0 |
| 3. | KF-6028 (*3) | 1.0 |
| 4. | Dimethicone 6CS | 5.0 |
| 5. | Cyclopentasiloxane | 5.0 |
| 6. | Isotridecyl isononanoate | 4.0 |

(continued)

| | Formulation | % |
|---|---|---|
| 7. | SPD-T5 (*4) | 5.0 |
| 8. | SPD-Z5 (*5) | 35.0 |
| 9. | Sodium citrate | 0.2 |
| 10. | Sodium chloride | 0.5 |
| 11. | Dispersion of Example 9 | 20.0 |
| 12. | Preservative | proper |
| 13. | Fragrance | proper |
| 14. | Water | balance |
| | Total | 100.0 |

(*1) mixture of dimethicone 70-80% + (dimethicone/PEG-10/15) crosspolymer 20-30%, Shin-Etsu Chemical Co., Ltd.

(*2) mixture of cyclopentasiloxane 90-96% + (dimethicone/vinyl dimethicone) crosspolymer 4-10%, Shin-Etsu Chemical Co., Ltd.

(*3) PEG-9 polydimethylsiloxyethyl dimethicone, Shin-Etsu Chemical Co., Ltd.

(*4) dispersion of 40% fine particle of titanium oxide in cyclopentasiloxane, Shin-Etsu Chemical Co., Ltd.

(*5) dispersion of 60% fine particle of zinc oxide in cyclopentasiloxane, Shin-Etsu Chemical Co., Ltd.

(Preparation method)

A: Uniformly mixing ingredients 1 to 6.
B: Uniformly mixing ingredients 9 to 14.
C: With stirring, adding C to B, emulsifying, adding ingredients 7 and 8 to prepare a W/O sunscreen milky lotion.

The W/O sunscreen milky lotion thus obtained showed high stability, high transparency, a non-sticky pleasant feeling on use, and water resistance. Ingredient 11 was highly stable and easy to handle and admit.

[Example 28] O/W sunscreen milky lotion

| | Formulation | % |
|---|---|---|
| 1. | MT-200ST (*1) | 10 |
| 2. | BG | 2 |
| 3. | KF-6100 (*2) | 1.4 |
| 4. | KM-72 (*3) | 0.001 |
| 5. | Water | 6.599 |
| 6. | Cyclopentasiloxane | 10 |
| 7. | KF-56A (*4) | 3 |
| 8. | Cetyl alcohol | 0.5 |
| 9. | Polyoxyethylene sorbitan monoleate | 2.5 |
| 10. | Glyceryl stearate (SE) | 0.5 |
| 11. | KF-6011 (*5) | 1 |
| 12. | DPG | 6 |
| 13. | BG | 6 |
| 14. | Carboxyvinyl polymer | 0.3 |
| 15. | Acrylic polymer | 0.3 |
| 16. | Methyl paraben | 0.2 |
| 17. | Phenoxyethanol | 0.3 |
| 18. | EDTA-2Na | proper |

(continued)

| | Formulation | % |
|---|---|---|
| 19. | Water | balance |
| 20. | 10% aqueous solution of sodium hydroxide | proper |
| | Total | 100.0 |

(*1) stearic acid-treated fine particle of titanium oxide
(*2) polyglyceryl-3 disiloxane dimethicone, Shin-Etsu Chemical Co., Ltd.
(*3) simethicone emulsion, Shin-Etsu Chemical Co., Ltd.
(*4) diphenylsiloxyphenyl trimethicone, Shin-Etsu Chemical Co., Ltd.
(*5) PEG-11 methyl ether dimethicone, Shin-Etsu Chemical Co., Ltd.

(Preparation method)

A: Uniformly dispersing ingredients 1 to 5 on a bead mill.
B: Uniformly mixing ingredients 6 to 8.
C: Uniformly mixing ingredients 9 to 19 and the dispersion of A.
D: Adding B to C, emulsifying, adding ingredient 20, and uniformly mixing.

The O/W sunscreen milky lotion thus obtained showed high stability, high transparency, a non-sticky pleasant feeling on use, and water resistance. The dispersion of A in the preparation method was highly stable and easy to handle and admit.

[Example 29] O/W primer

| | Formulation | % |
|---|---|---|
| 1. | TSPL-30-D5 (*1) | 3 |
| 2. | KSG-18A (*2) | 5 |
| 3. | KF-56A (*3) | 5 |
| 4. | Cyclopentasiloxane | 10 |
| 5. | ST-455FA (*4) | 10 |
| 6. | BG | 2 |
| 7. | KF-6100 (*5) | 1.6 |
| 8. | Water | 20 |
| 9. | Sodium chloride | 0.2 |
| 10. | BG | 10 |
| 11. | KF-6043 (*6) | 1.5 |
| 12. | Sodium acrylate-sodium acryloyldimethyltaurate copolymer (*7) | 1 |
| 13. | (acrylate/alkyl (C10-30) acrylate) crosscopolymer (2% aqueous solution) | 20 |
| 14. | Arginine (10% aqueous solution) | proper |
| 15. | EDTA-2Na (10% aqueous solution) | 0.1 |
| 16. | Water | balance |

(continued)

| Formulation | % |
|---|---|
| Total | 100.0 |

(*1) solution of 30% trimethylsiloxysilylcarbamoyl pullulan) in cyclopentasiloxane, Shin-Etsu Chemical Co., Ltd.
(*2) mixture of diphenylsiloxyphenyl trimethicone 80-90% + (dimethicone/phenyl vinyl dimethicone) crosspolymer 10-20%, Shin-Etsu Chemical Co., Ltd.
(*3) diphenylsiloxyphenyl trimethicone, Shin-Etsu Chemical Co., Ltd.
(*4) stearic acid-treated fine particle of titanium oxide
(*5) polyglyceryl-3 disiloxane dimethicone, Shin-Etsu Chemical Co., Ltd.
(*6) PEG-10 dimethicone, Shin-Etsu Chemical Co., Ltd.
(*7) Simulgel EG, Seppic

(Preparation method)

A: Uniformly mixing ingredients 1 to 4,
B: Uniformly dispersing ingredients 5 to 9 on a paint shaker,
C: Uniformly mixing ingredients 10 to 16,
D: Adding A to C and mixing with B to prepare an O/W primer.

The O/W primer thus obtained showed high stability, high transparency, a non-sticky pleasant feeling on use, and water resistance. The dispersion of B in the preparation method was highly stable and easy to handle and admit.

**Claims**

1.  A dispersion comprising components (a) to (c):

    (a) 10 to 70% by weight of a hydrophobized fine particle of inorganic powder having a number average primary particle diameter of 10 to 200 nm, as determined by image analysis of a transmission electron photomicrograph,
    (b) 1.0 to 30% by weight of an aqueous component having at least one alcoholic hydroxy group, and
    (c) 1.0 to 20% by weight of a polyglycerin-modified silicone which dissolves in component (b).

2.  The dispersion of claim 1 wherein the fine particle of inorganic powder as component (a) has a UV-shielding effect.

3.  The dispersion of claim 2 wherein the fine particle of inorganic powder as component (a) is titanium oxide.

4.  The dispersion of claim 1 or 2 wherein component (a) is a fine particle of inorganic powder which has been hydrophobized with at least one agent selected from stearic acid, isostearic acid and triethoxycaprylylsilane.

5.  The dispersion of claim 1 wherein component (b) is a compound having two alcoholic hydroxy groups in the molecule.

6.  The dispersion of claim 1 wherein component (c) is polyglyceryl-3 disiloxane dimethicone.

7.  The dispersion of claim 1 wherein the ratio of the amount of component (c) to the amount of component (b), (c)/(b), is from 0.35/1 to 1.0/1.

8.  The dispersion of claim 1, further comprising (d) water.

9.  The dispersion of claim 8 wherein the content of component (d) is 8 to 82% by weight of the dispersion.

10. A cosmetic composition comprising the dispersion of any one of claims 1 to 9.

## INTERNATIONAL SEARCH REPORT

| International application No. |
|---|
| **PCT/JP2023/021486** |

### A. CLASSIFICATION OF SUBJECT MATTER

*A61Q 1/00*(2006.01)i; *A61Q 17/04*(2006.01)i; *A61Q 19/00*(2006.01)i; *A61K 8/04*(2006.01)i; *A61K 8/19*(2006.01)i; *A61K 8/34*(2006.01)i; *A61K 8/893*(2006.01)i

FI: A61K8/04; A61K8/893; A61K8/19; A61K8/34; A61Q19/00; A61Q17/04; A61Q1/00

According to International Patent Classification (IPC) or to both national classification and IPC

### B. FIELDS SEARCHED

Minimum documentation searched (classification system followed by classification symbols)

A61Q1/00; A61Q17/04; A61Q19/00; A61K8/04; A61K8/19; A61K8/34; A61K8/893

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2010-159229 A (TOKIWA CORP) 22 July 2010 (2010-07-22) example 1, claims, paragraphs [0019], [0049] | 1-10 |
| X | JP 2006-213619 A (FANCL CORP) 17 August 2006 (2006-08-17) example 4, claims | 1-10 |
| X | JP 2009-084232 A (POLA CHEM IND INC) 23 April 2009 (2009-04-23) example 1, production example 1, claims, paragraphs [0013], [0016], [0022], [0026], [0029] | 1-10 |
| X | JP 2007-269756 A (KOSE CORP) 18 October 2007 (2007-10-18) example 3, claims, paragraphs [0014], [0028] | 1-2, 5-10 |
| A | | 3-4 |
| P, X | JP 2023-056931 A (KAO CORP) 20 April 2023 (2023-04-20) examples, claims | 1-10 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 August 2023** | **22 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/021486**

| Patent document cited in search report | | | Publication date (day/month/year) | Patent family member(s) | Publication date (day/month/year) |
|---|---|---|---|---|---|
| JP | 2010-159229 | A | 22 July 2010 | US 2010/0172850 A1<br>example 1, claims, paragraphs [0019], [0048]<br>CN 101773455 A | |
| JP | 2006-213619 | A | 17 August 2006 | (Family: none) | |
| JP | 2009-084232 | A | 23 April 2009 | (Family: none) | |
| JP | 2007-269756 | A | 18 October 2007 | (Family: none) | |
| JP | 2023-056931 | A | 20 April 2023 | (Family: none) | |

Form PCT/ISA/210 (patent family annex) (January 2015)

**REFERENCES CITED IN THE DESCRIPTION**

**Patent documents cited in the description**

- JP 2006001886 A
- WO 2016178380 A
- JP 2014201569 A
- JP 2007016111 A
- JP H07247119 A